Europäisches Patentamt

European Patent Office

Office européen des brevets

(19)

(11) Numéro de publication: **0 022 697**
**B1**

(12) **FASCICULE DE BREVET EUROPEEN**

(45) Date de publication du fascicule de brevet: **30.12.81**

(51) Int. Cl.³: **C 07 C 149/14**

(21) Numéro de dépôt: **80400951.2**

(22) Date de dépôt: **25.06.80**

(54) **Procédé de préparation directe d'aldéhyde de bêta-méthylthio-propionique.**

(30) Priorité: **10.07.79 FR 7917827**

(43) Date de publication de la demande:
**21.01.81 Bulletin 81/3**

(45) Mention de la délivrance du brevet:
**30.12.81 Bulletin 81/52**

(84) Etats Contractants Désignés:
**BE CH DE FR GB IT LI NL SE**

(56) Documents cités:
**DE - A - 2 320 544**
**FR - A - 1 520 328**
**FR - A - 1 526 355**
**FR - A - 2 314 917**
**US - A - 2 626 282**

(73) Titulaire: **RHONE-POULENC INDUSTRIES**
**Brevets Pharma 25 Quai Paul Doumer**
**F-92408 Courbevoie Cedex (FR)**

(72) Inventeur: **Komorn, Yves**
**19, avenue Joannes Hubert**
**F-69160 - Tassin-La-Demi-Lune (FR)**
Inventeur: **Schwachhofer, Ghislain**
**Chemin de Belmont Le mas Rillier**
**F-01700 - Miribel (FR)**

(74) Mandataire: **Cazes, Jean-Marie et al,**
**RHONE POULENC Service Brevets Chimie et**
**Polymères B.P. 753**
**F-75360 Paris Cedex 08 (FR)**

Courier Press, Leamington Spa, England.

# 0 022 697

## Procédé de préparation directe d'aldéhyde de
## bêta-méthylthio-propionique

La présente invention concerne un procédé de préparation directe d'aldéhyde béta-méthylthiopropionique. Elle concerne plus particulièrement un perfectionnement à la préparation d'aldéhyde béta-méthylthiopropionique par réaction de méthylmercaptan et d'un mélange gazeux contenant de l'acroléine résultant de l'oxydation catalytique du propylène par l'air en présence d'eau.

On connaît, dans l'art antérieur, le brevet français 75.20183, publié sous le numéro 2 314 917 qui décrit un procédé de préparation d'aldéhyde béta-méthylthiopropionique à partir de méthylmercaptan et d'un mélange gazeux contenant de l'acroléine résultant de l'oxydation catalytique du propylène. Selon ce procédé, dans un premier stade le mélange gazeux brut issu du réacteur d'oxydation est envoyé dans une colonne d'absorption où l'acide acrylique contenu dans le mélange gazeux est éliminé; dans un second stade, le mélange gazeux ne contenant plus d'acide acrylique est condensé de façon à éliminer l'eau qu'il contient. Après cette double élimination de l'acide acrylique et de l'eau, le mélange gazeux contenant l'acroléine absorbée dans l'aldéhyde béta-méthylthiopropionique est mis en réaction avec du méthylmercaptan pour donner de l'aldéhyde béta-méthylthiopropionique.

Le principal inconvénient de ce type de procédé est que lors de l'opération de condensation destinée à éliminer l'eau contenue dans le mélange gazeux, il est matériellement impossible de ne pas condenser simultanément une partie de l'acroléine contenue dans le mélange gazeux. On obtient alors une solution aqueuse contenant de 10 à 20% de l'acroléine formée lors de l'oxydation du propylène.

Il devient nécessaire pour ne pas perdre cette quantité appréciable d'acroléine de la séparer de la solution aqueuse préférentiellement par distillation et de la réintroduire dans le troisième stade évoqué plus haut c'est-à-dire au stade de la réaction du méthylmercaptan avec l'acroléine.

Cette séparation et cette réintroduction impliquent un isolement donc la présence d'une masse d'acroléine pratiquement pure, ce qui contrarie l'esprit général du procédé qui a pour but de préparer l'aldéhyde béta-méthylthiopropionique sans isoler intermédiairement l'acroléine. Ceci est, en effet, d'une grande importance pratique, surtout à l'échelle industrielle, car l'homme de l'art sait bien que l'acroléine est un produit toxique dont la manipulation présente de nombreux risques et qui a été la source de nombreux accidents très dommageables pour l'environnement.

De plus, la réintroduction d'un flux d'acroléine concentrée annexe au flux gazeux dilué dans l'étape de réaction avec le méthylmercaptan est rendue complexe par le fait que selon la demande de brevet mentionnée ci-dessus, il est impératif d'opérer à une valeur de la stoechiométrie acroléine méthylmercaptan très stricte : en effet, la concentration d'hémithioacétal doit être en permanence comprise entre 0 et 1%, la valeur 0 étant exclue.

La présente invention a pour but de supprimer cette étape où l'acroléine se trouve pratiquement pure sous forme liquide tout en obtenant un rendement élevé en aldéhyde béta-méthylthiopropionique par rapport à l'acroléine contenue dans le courant gazeux brut, issu du réacteur d'oxydation du propylène.

L'invention a donc pour objet un procédé de préparation directe d'aldéhyde béta-méthylthiopropionique selon lequel, dans une première étape, on élimine l'acide acrylique contenu dans le courant gazeux brut issu de la réaction d'oxydation catalytique du propylène par l'air par absorption dudit courant gazeux brut dans l'eau ou dans un solvant; dans une deuxième étape, on élimine l'eau contenue dans le flux gazeux issu de la première étape par condensation dudit flux; et, dans une troisième étape, on met en réaction le mélange gazeux issu de la deuxième étape avec du méthylmercaptan pour former l'aldéhyde béta-méthylthiopropionique, caractérisé en ce que l'on vaporise partiellement le mélange liquide issu de la deuxième étape contenant principalement de l'eau et de l'acroléine de façon à obtenir une phase gazeuse contenant pratiquement toute l'acroléine et une phase liquide ne contenant pratiquement pas d'acroléine et en ce que l'on recycle ladite phase gazeuse contenant pratiquement toute l'acroléine dans ledit flux gazeux issu de la première étape d'absorption, avant la deuxième étape de condensation.

L'oxydation catalytique du propylène par l'air peut être effectuée selon les divers procédés bien connus dans l'art antérieur. On peut, en particulier, faire passer dans un réacteur tubulaire, contrôlé en température par un fluide caloporteur, contenant un catalyseur à base d'oxydes de cobalt, de molybdène, de fer et de bismuth (un tel catalyseur est décrit dans la demande de brevet français 76.27531 publiée sous le numéro 2 364 061) un mélange de propylène, d'air et d'eau.

Quel que soit le procédé utilisé, le courant gazeux issu du réacteur d'oxydation contient comme principaux produits condensables de l'acroléine, de l'eau et de l'acide acrylique.

L'eau et l'acide acrylique doivent être éliminés pour éviter les réactions de dégradation qui conduisent à une diminution considérable du rendement en aldéhyde béta-méthylthiopropionique.

Pour éliminer l'acide acrylique, on peut faire passer, d'une manière connue en soi, le courant gazeux issu du réacteur d'oxydation dans une colonne d'absorption alimentée en eau (comme décrit dans le brevet français 1 393 175) ou par un solvant tel que le tributylphosphate (comme cela est décrit dans le brevet français 1 558 432) ou le mélange biphényle et oxyde de phényle par exemple (brevet français 2 146 386).

2

**0 022 697**

La solution d'acide acrylique peut être ensuite traitée pour récupérer l'acide acrylique pur comme décrit dans la demande de brevet européen n° 110.

Cette opération d'élimination de l'acide acrylique étant effectuée, on élimine l'eau, d'une façon également connue en soi, en condensant à basse température le flux gazeux issu de la colonne d'absorption précédente. La température de sortie du ou des condenseurs utilisés est avantageusement comprise entre 0 et 10°C.

Selon une première caractéristique de l'invention, le mélange liquide issu de l'étape de condensation, mélange qui contient essentiellement de l'eau et de l'acroléine, est soumis à une vaporisation partielle. On entend par "vaporisation partielle", au sens de la présente invention, une séparation en deux phases: une phase aqueuse ne contenant plus d'acroléine et une phase gazeuse contenant la totalité de l'acroléine qui a été condensée dans l'étape de condensation.

Selon une deuxième caractéristique de l'invention, la phase gazeuse obtenue comme décrit ci-dessus est recyclée dans le flux gazeux issu de la première étape d'absorption de l'acide acrylique avant son entrée dans le ou les condenseurs.

On effectue préférentiellement la vaporisation partielle à une température comprise entre environ 70°C et environ 130°C sous une pression comprise entre environ 0,8 bar et environ 3 bars absolus.

Selon un mode de réalisation particulier de l'invention, on effectue la vaporisation partielle du mélange liquide obtenu après condensation dans un appareil de "distillation-flash" bien connu en lui-même qui assure avec un temps de séjour court une distillation correspondant à un seul étage théorique.

Selon un deuxième mode de réalisation particulier de l'invention, on effectue la vaporisation partielle du mélange liquide dans un appareil de distillation d'épuisement c'est-à-dire dans une colonne de distillation où l'alimentation liquide se fait dans la partie supérieure, les étages inférieurs épuisant le mélange en acroléine.

Selon un troisième mode de réalisation particulier de l'invention, on utilise un appareil de stripping par un gaz. On peut, par exemple, alimenter le mélange liquide en tête d'une colonne au bas de laquelle on introduit un gaz chauffé (azote ou vapeur d'eau par exemple).

Selon un quatrième mode de réalisation particulier de l'invention, on peut utiliser un évaporateur à couche mince, rotatif ou non.

Il est clair pour l'homme de l'art que l'invention s'étend également au cas où on utilise en série toute combinaison des quatres dispositifs décrits ci-dessus.

Selon l'invention, on obtient donc en sortie de l'appareil de vaporisation partielle, une solution aqueuse ne contenant plus d'acroléine. On a ainsi évité tout stockage et toute manipulation d'acroléine sous forme concentrée et liquide.

Le mélange gazeux issu du ou des condenseurs est en-suite mis en réaction, d'une façon connue en soi, avec le méthylmercaptan. On peut opérer comme décrit par exemple dans la demande de brevet français 75.20183 précitée, c'est-à-dire en opérant comme suit: on introduit le mélange gazeux issu du ou des condenseurs en bas d'une colonne d'absorption alimentée en tête par de l'aldéhyde béta-méthylthiopropionique. Le mélange de sortie de cette colonne (acroléine absorbée dans l'aldéhyde béta-méthylthiopropionique) arrive dans un réacteur où sont introduits le méthylmercaptan et le catalyseur (triéthylamine par exemple). Une partie de l'aldéhyde béta-méthylthiopropionique produit est soutirée, l'autre étant après refroidissement recyclée en tête de la colonne d'absorption d'où sont éliminés au sommet les gaz résiduaires. Selon la demande 75.20183, on maintient dans le milieu réactionnel une concentration en hémithioacétal intermédiaire comprise entre 0 (0 exclu) et 1%.

Il est clair que l'invention s'applique au cas où le mélange gazeux issu du ou des condenseurs serait mis en réaction avec le méthylmercaptan selon un procédé différent de celui qui vient d'être décrit.

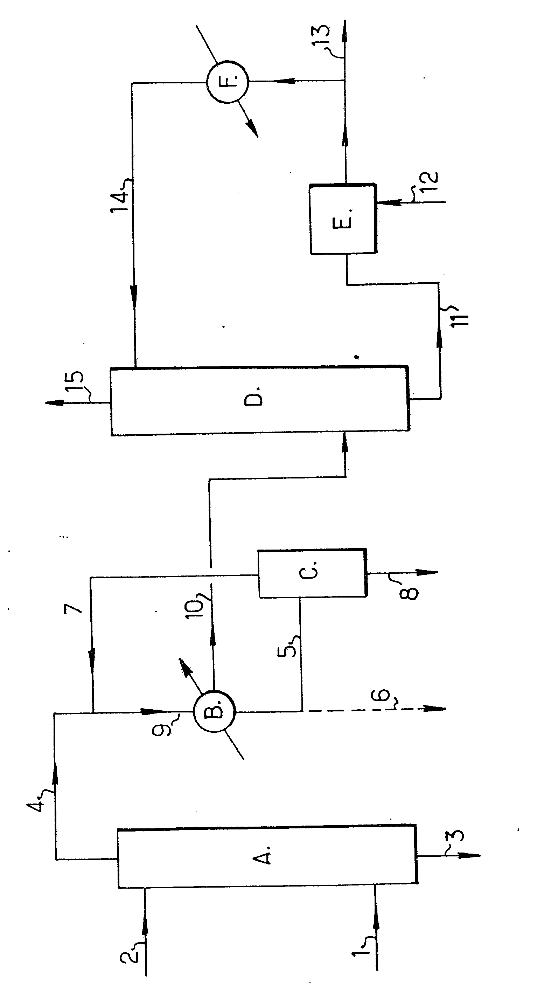

Les exemples suivants qui se réfèrent à la figure jointe illustrent plus complètement l'invention. Il va sans dire que ces exemples ne sauraient en aucune façon constituer une limitation de l'invention.

Exemple 1

Cet exemple illustre l'invention sur une opération isolée de vaporisation partielle à partir d'un condensat initial.

L'effluent gazeux 1 provenant de la réaction d'oxydation catalytique du propylène par l'air en présence d'eau a la composition suivante (en moles):

| | |
|---|---|
| — gaz inertes | 547 |
| — eau | 395 |
| — acroléine | 49,6 |
| — acide acrylique | 6 |
| — acétaldéhyde et divers | 2,4 |

3

**0 022 697**

Cet effluent qui est à une température de 350°C est envoyé dans la colonne d'absorption A alimentée en 2 par du tributylphosphate à une température de 50°C. On recueille en 3 une solution d'acide acrylique et de tributylphosphate contenant des traces d'eau et d'acroléine.

Le flux gazeux 4 qui sort en tête de la colonne A à 80°C a la composition suivante:

| | |
|---|---|
| — gaz inertes | 547 |
| — eau | 392 |
| — acroléine | 49,2 |
| — acide acrylique | 0,06 |
| — acétaldéhyde et divers | 2 |

et est introduit dans un condenseur B où il est refroidi à 0°C.

Selon l'art antérieur le mélange liquide issu du condenseur était récupéré en 6 et traité séparément afin de récupérer l'acroléine qu'il contenait. Le mélange gazeux 10 était envoyé directement à l'étape de réaction avec le méthylmercaptan. Les compositions de ces deux mélanges sont données dans le tableau 1 suivant:

TABLEAU 1

| | 6 | 10 |
|---|---|---|
| — gaz inertes | 0 | 547 |
| — eau | 389,7 | 2,3 |
| — acroléine | 7,2 | 42 |
| — acide acrylique | 0,06 | 0 |
| — acétaldéhyde et divers | 0,5 | 1,5 |

Le perfectionnement apporté par le procédé selon l'invention consiste à introduire le mélange liquide 6 par la ligne 5 dans l'appareil C constitué ici par deux étages de distillation flash travaillant respectivement à des températures de 97° et de 100°C sous pression atmosphérique.

Cette vaporisation partielle en 2 étages fournit d'une part un ensemble de deux phases gazeuses qui, réunies, forment le mélange 7 destiné à être recyclé en amont du condenseur B, et d'autre part une phase liquide 8 aqueuse ne contenant que des traces d'acroléine, d'acide acrylique et de divers, selon les compositions données dans le tableau 2:

TABLEAU 2

| | 6 | 7 | 8 |
|---|---|---|---|
| — gaz inertes | 0 | 0 | 0 |
| — eau | 389,7 | 102,5 | 287,2 |
| — acroléine | 7,1 | 7,15 | 0,05 |
| — acide acrylique | 0,06 | — | 0,06 |
| — acétaldéhyde et divers | 0,5 | — | 0,5 |

Si on conduit simultanément dans un processus continu la condensation, la vaporisation partielle et le recyclage en amont du condenseur B, il est clair qu'un équilibre s'établira autour d'une quantité permanente et constante d'acroléine condensée puis revaporisée plus concentrée tandis que la quasi totalité de l'acroléine produite et arrivant par la ligne 4 demeurera à l'état gazeux dans le flux de sortie 10 allant à la réaction avec le méthylmercaptan.

De plus selon l'invention, il n'y a plus d'acroléine à séparer sous forme liquide et concentrée, exigeant une mise en réaction complémentaire avec le méthylmercaptan. En effet, selon l'art antérieur, l'acroléine condensée avec l'eau était récupérée de façon conventionnelle, c'est-à-dire généralement par distillation, ce qui fournissait un azéotrope qui est constitué par de l'acroléine contenant environ 3% d'eau; cette acroléine concentrée pouvait être mise à son tour en réaction avec le méthylmercaptan.

### Exemple 2

On opère en continu comme dans l'exemple 1, mais l'appareil C est ici une colonne d'épuisement alimentée sur le plateau supérieur qui est à 83°C sous une pression de 1,5 atmosphère absolue.

Le mélange gazeux 10 est introduit dans une colonne d'absorption D alimentée en tête avec de l'aldéhyde béta-méthylthiopropionique. L'acroléine absorbée dans l'aldéhyde béta-méthylthiopropionique est introduite dans le réacteur E où sont également introduits le catalyseur (triéthylamine, 0,05% de la masse réactionnelle) et le méthylmercaptan (débit molaire de 49,3 moles). En sortie du réacteur, on soutire en 13 une partie de l'aldéhyde béta-méthylthiopropionique produit alors que la partie en excès initiale 14 est recyclée après refroidissement (F) à une température de —10°C en tête de la colonne 14 de laquelle s'échappe en 15 les gaz résiduaires.

Les débits molaires des différentes lignes sont donnés le tableau 3 suivant:

TABLEAU 3

| composition (en moles) | 1 | 4 | 9 | 5 | 7 | 8 | 10 | 15 | 13 |
|---|---|---|---|---|---|---|---|---|---|
| Inertes | 547 | 547 | 547 | | | | 547 | 547 | |
| Eau | 395 | 392 | 398 | 396 | 6 | 390 | 2 | — | 2 |
| Acroléine | 49,6 | 49,2 | 59,9 | 10,75 | 10,7 | 0,05 | 49,15 | — | — |
| Acide acrylique | 6 | 0,06 | 0,06 | 0,06 | — | 0,06 | — | — | — |
| Acétal-déhyde et divers | 2,4 | 2 | 2,2 | 0,7 | 0,2 | 0,5 | 1,5 | 1,5 | 0,4 |
| Aldéhyde béta-méthylthio-propionique | — | — | — | — | — | — | — | 0,15 | 48,7 |

**0 022 697**

On constate que 99,5% de l'acroléine engagée par la ligne 4 est recueillie à l'état vapeur dans la ligne 10, dans le mélange gazeux introduit dans la colonne d'absorption D.

La comparaison de la composition de la ligne 10 du tableau 1 de l'exemple 1 (art antérieur) et de la composition de la ligne 10 du tableau 3 ci-dessus (invention) montre clairement l'avantage apporté par le procédé de l'invention (42 moles d'acroéine gazeuse sont présentes dans le courant 10 de l'art antérieur alors que 49,2 le sont dans le courant 10 de l'invention pour la même quantité d'acroléine présente dans la ligne 4).

L'aldéhyde béta-méthylthiopropionique obtenu selon le procédé de l'invention est utile, notamment, en tant que produit intermédiaire pour la synthèse de la méthionine utilisée pour l'alimentation animale.

**Revendications**

1. Procédé de préparation directe d'aldéhyde béta-méthylthiopropionique selon lequel, dans une première étape, on élimine l'acide acrylique contenu dans le courant gazeux brut issu de la réaction d'oxydation catalytique du propylène par l'air par absorption dudit courant gazeux brut dans l'eau ou dans un solvant; dans une deuxième étape, on élimine l'eau contenue dans le flux gazeux issu de la première étape par condensation dudit flux; et dans une troisième étape, on met en réaction le mélange gazeux issu de la deuxième étape avec du méthylmercaptan pour former l'aldéhyde béta-méthylthiopropionique, caractérisé en ce que l'on vaporise partiellement le mélange liquide issu de la deuxième étape contenant principalement de l'eau et de l'acroléine de façon à obtenir une phase gazeuse contenant pratiquement toute l'acroléine et une phase liquide ne contenant pratiquement pas d'acroléine et en ce que l'on recycle ladite phase gazeuse contenant pratiquement toute l'acroléine dans ledit flux gazeux issu de la première étape d'absorption, avant la deuxième étape de condensation.

2. Procédé selon la revendication 1 caractérisé en ce que l'on vaporise partiellement ledit mélange liquide issu de ladite deuxième étape à une température comprise entre 70°C environ et 130°C environ.

3. Procédé selon la revendication 1 caractérisé en ce que l'on vaporise partiellement ledit mélange liquide issu de ladite deuxième étape à une pression comprise entre environ 0,8 bars et environ 3 bars.

**Claims**

1. Process for the direct preparation of beta-methylthiopropionaldehyde, in accordance with which, in a first step, the acrylic acid contained in the crude stream of gas produced by the catalytic oxidation reaction of propylene with air is removed by absorption of the said crude stream of gas in water or in a solvent; in a second step, the water contained in the flow of gas originating from the first step is removed by condensation of the said flow; and in a third step, the gaseous mixture originating from the second step is reacted with methylmercaptan to form beta-methylthiopropionaldehyde, characterised in that the liquid mixture originating from the second step, which contains mainly water and acrolein, is partially vaporised so as to obtain a gaseous phase containing virtually all the acrolein and a liquid phase containing virtually no acrolein, and in that the said gaseous phase containing virtually all the acrolein is recycled into the said flow of gas originating from the first absorption step, before the second condensation step.

2. Process according to Claim 1, characterised in that the said liquid mixture originating from the said second step is partially vaporised at a temperature between about 70°C and about 130°C.

3. Process according to Claim 1, characterised in that the said liquid mixture originating from the said second step is partially vaporised at a pressure between about 0.8 bar and about 3 bars.

**Patentansprüche**

1. Verfahren zur direkten Herstellung von β-Methylthiopropionaldehyd, wobei man in einer ersten Stufe de in dem Rohgasstrom der katalytischen Oxidationsreaktion von Propylen mit Luft enthaltene Acrylsäure durch Absorption in Wasser oder in einem Lösungsmittel aus dem Rohgasstrom abtrennt, in einer zweiten Stufe das in dem Gasstrom der ersten Stufe enthaltene Wasser durch Kondensation eliminiert; und in einer dritten Stufe das Gasgemisch der zweiten Stufe mit Methylmercaptan zur Reaktion bringt, um β-Methylthiopropionaldehyd zu bilden, dadurch gekennzeichnet, daß man teilweise das aus der zweiten Stufe stammende flüssige Gemisch, das hauptsächlich Wasser und Acrolein enthält, derart verdampft, daß man eine Gasphase, die praktisch das gesamte Acrolein und eine flüssige Phase, die praktisch kein Acrolein aufweist, erhält, und daß man die genannte, praktisch das gesamte Acrolein des aus der ersten Absorptionsstufe stammenden Gasstroms enthaltende Gasphase vor die zweite Kondensationsstufe zurückführt.

2. Verfahren nach Anspruch 1, dadurch gekennzeichnet, daß man das genannte, aus der zweiten Stufe stammende flüssige Gemisch bei einer Temperatur zwischen etwa 70 und etwa 130°C teilweise verdampft.

3. Verfahren nach Anspruch 1, dadurch gekennzeichnet, daß man das aus der zweiten Stufe stammende flüssige Gemisch bei einem Druck zwischen etwa 0,8 und 3 bar teilweise verdampft.

7

1